# EUROPEAN PATENT APPLICATION

(11) **EP 3 199 468 A1**
(43) Date of publication of application: **02.08.2017**
(21) Application number: 17151309.6
(22) Date of filing: 13.01.2017
(51) Int. Cl.: B65D 33/00, G01K 11/12, A61N 1/04, A61N 1/08

(54) **ELECTRODES CONTAINER**

(30) Priority: 29.01.2016 IT UB20160164
(71) Applicant: Fiab S.P.A., 50039 Vicchio (Firenze) (IT)
(72) Inventor: FASANO, Antonio, 50139 Firenze (IT); CALABRO', Alberto, 50139 Firenze (IT)
(74) Representative: Paparo, Aldo

(57) **Abstract**

Described is a container (1) for electrodes (E) to be used preferably in apparatuses for stimulating the human body and in the health sector, which allows a signal to be sent to the user upon the reaching, by an inside environment (3) of the container, of at least one temperature threshold, the reaching of which can produce negative effects on the components of the product which are perishable by heat, in such a way as to adversely affect the correct operation before the nominal expiry date indicated.

## Description

This invention relates to a container for electrodes to be used preferably in apparatuses for stimulating the human body and in the health sector, which allows a signal to be sent to the user, in an irreversible manner, upon the reaching, by an environment inside the container, of at least one temperature threshold, the reaching of which can produce negative effects on the components of the electrode in such a way as to adversely affect the correct operation before the nominal expiry date indicated. Currently, each electrode, at the time of sale of the electrode or of the medical apparatus in which it is built-in, is packaged in a respective container. The container is usually of the bag type and made with at least one material suitable for keeping its inside environment sterile, where at least one electrode is situated. The material may be, for example, a plastic material. The container is configured to keep the electrode in a sterile environment, and the dealer, in accordance with the law, must guarantee and/or declare a certain working life of the electrode, which starts at the time of production and/or packaging of the electrode, which is also declared by the dealer, and ends upon the opening of the container by the user.

In accordance with the law, the dealer must guarantee that the deterioration of the electrode, during the working life, is zero or in any case very limited, and such as not to adversely affect the correct and safe operation of the electrode.

The guarantee provided by the dealer concerning the working life is based on the electrode being maintained within a certain temperature range. Therefore, the dealer guarantees that the electrode remains within certain qualitative conditions, and in particular suitable for the use, for a certain working life, but only if the temperature of an environment inside the container remains within a certain temperature range, which is commonly called the "operating range".

For example, the duration of the working life guaranteed by the dealer may be thirty months, and the operating range be from zero to fifty degrees.

If the temperature leaves the operating range, the working life of the packaged electrode decreases or is even reduced to zero, and therefore the user cannot know, even if a time equal to the working life has not yet past since its production, whether the electrode is actually damaged or in any case the conditions of the electrode are no longer those guaranteed by the dealer.

The aim of this invention is to provide a container for packaging electrodes to be used preferably in apparatuses for stimulating the human body and in the health sector, which is able to signal to the user a possible deterioration of the conditions of the electrode due to the electrode leaving the operating range guaranteed by the dealer.

This aim is achieved by means of a sterile container for packaging at least one electrode suitable for preferably medical applications, comprising a heat-sensitive element configured to be irreversibly activated when an inside environment of the container reaches at least one temperature threshold, in such a way as to be able to permanently indicate the reaching of this temperature threshold to a user who observes the container from the outside.

This temperature threshold may be defined, for convenience, as the first threshold.

This element is configured to be activated when reaching the first threshold from temperatures lower than the first threshold or from temperatures higher than the first threshold.

For example, the first threshold could be zero degrees Celsius, equivalent to 273.15 degrees Kelvin, and the element could be configured to activate when the temperature inside the container touches zero degrees Celsius arriving from temperatures greater than zero degrees Celsius. Alternatively, as another example, the first threshold could be fifty degrees Celsius, equivalent to 323.15 degrees Kelvin, and the element could be configured to activate when the temperature inside the container touches fifty degrees Celsius arriving from temperatures lower than fifty degrees Celsius.

The element may also be advantageously configured to be irreversibly activated when the inside environment reaches a further temperature threshold, in such a way as to be able to permanently indicate the reaching of this further threshold to a user who observes the container from the outside.

This further threshold may be defined, for convenience, as the second threshold.

The element is advantageously configured to be activated irreversibly when reaching the first temperature threshold from temperatures higher than the first threshold, and to be activated irreversibly when reaching the second temperature threshold from temperatures lower than the second threshold.

In the latter case, therefore, the first threshold and the second threshold define a temperature range which is felt by the heat-sensitive element, which may correspond to the operating range guaranteed by the dealer.

In effect, since the element is activated if the first threshold is reached from temperatures greater than the first threshold, or the second threshold is reached from temperatures lower than the second threshold, it is clear that the first threshold may be the lowest value of the operating range and the second threshold may be the highest value of the operating range. Preferably, the heat-sensitive element forms part of at least a portion of the container configured in such a way that the act of activating or the activation of the element is visible from the outside of the container.

The heat-sensitive element preferably comprises an ink or a paint sensitive to temperature and/or to heat.

The heat-sensitive element is preferably situated on an inner surface of the container.

The heat-sensitive element is fixed and/or attached to the remaining part of the container in such a way as to remain at least partly in the inside environment defined by the container.

The heat-sensitive element could define at least part of a wall of the container.

The heat-sensitive element might contribute to define and/or to delimit the inside environment.

According to another aspect, this invention relates to an apparatus for medical applications comprising a container having one or more of the above-mentioned features.

The features of this invention are described in detail below by way of a non-limiting example of the more general concepts claimed.

The detailed description which follows relates to the accompanying drawings, in which:
- Figure 1 shows a cross section of a container according to a possible embodiment of the invention and an electrode packaged in the container, in a situation wherein the temperature of the electrode has never left the operating range of the electrode;
- Figure 2 shows the container and electrode in a situation such that the temperature of the electrode has at least once left the operating range or has reached at least once one of the ends of the operating range;
- Figure 3 is a perspective view of the container and electrode in the situation of Figure 1.

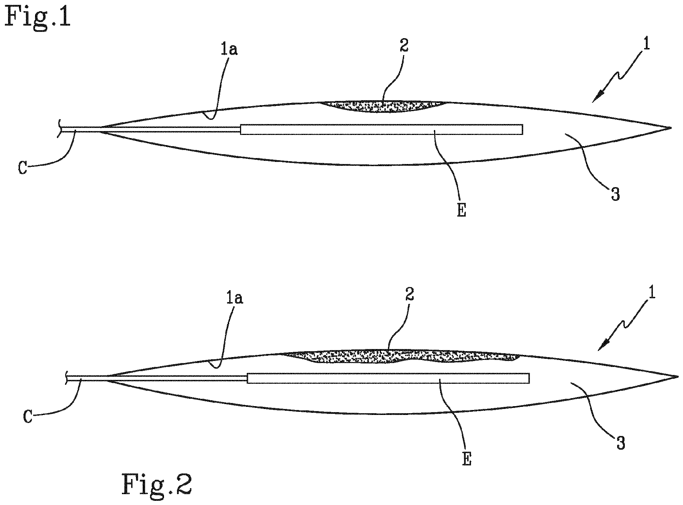

Figures 1 and 2 shows a cross section of a sterile container 1 for packaging at least one electrode E. The electrode E is suitable for use in medical apparatuses, such as, for example, apparatuses for electrotherapy or electrosurgical or electro-physiotherapy operations.

The container 1 comprises a heat-sensitive element 2 configured to activate irreversibly when the temperature of an environment 3 inside the container 1 reaches at least one threshold temperature. For this purpose, the heat-sensitive element 2 is situated at least partly in the inside environment 3.

If the container 1 were opaque (if, for example, is designed to contain photo-sensitive materials) the heat-sensitive element 2 can be advantageously located also outside the container.

The element 2 is configured to permanently signal the reaching of the threshold to a user who observes the container 1 from the outside.

In the situation of Figure 1 the container 2 is not activated since the temperature of the inside environment 3, felt by the element 2, has never left the operating range, from when the electrode E was packaged. In the situation of Figure 2 the container 2 is activated since the temperature of the inside environment 3 has at least one left the operating range or has at least once reached a lower end or an upper end of the range.

The heat-sensitive element 2 forms part of at least a portion of the container 1 configured in such a way that the act of activating or the activation of the element 2 is visible from the outside of the container 1. For example, the portion of the container 1 may be at least partly transparent.

The element 2 is also preferably situated on an inner surface 1 a of the container 1.

The element 2, in the embodiment illustrated, contributes to delimit and/or define the inside environment 3 of the container 1.

The element 2, in the embodiment illustrated, forms part of at least one wall of the container 1.

The heat-sensitive element 2 can comprise, for example, ink or paint sensitive to temperature and/or to heat.

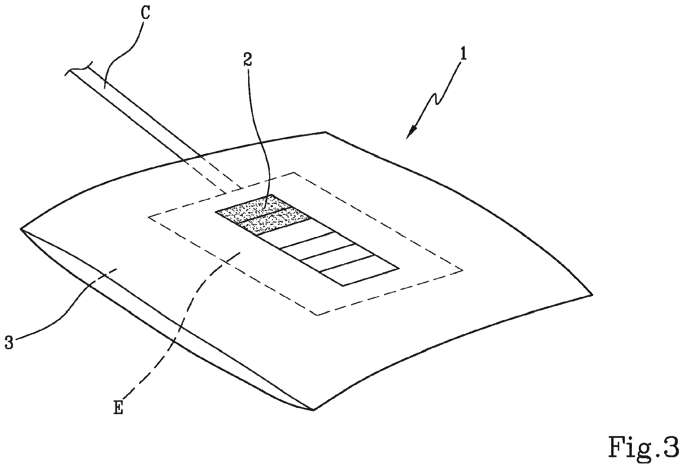

In the embodiment illustrated in Figures 1 and 2, the sensitive element 2 comprises a portion of ink which explodes, as can be seen by comparing, precisely, Figure 1 with Figure 2, when the temperature of the inside environment 3 felt by element 2 reaches or exceeds that threshold value. In other embodiments, the element may consist of a substance with a permanent thermal colour change, such as, for example shown in Figure 3, in other words the heat-sensitive element 2 may be configured as a thermometric scale in which the reaching of the maximum and minimum temperatures remains permanently indicated.

The heat-sensitive element 2 is advantageously configured to be activated when reaching the at least one temperature threshold from temperatures lower than the threshold or from temperatures higher than the threshold. The threshold temperature, which may be called first threshold temperature, may be a lower or upper end of the operating range of the electrode E.

The heat-sensitive element 2 is advantageously configured to be irreversibly activated when the inside environment 3 also reaches a further temperature threshold, in such a way as to be able to permanently indicate the reaching of this further threshold to a user who observes the container 1 from the outside.

This further threshold may be defined, for convenience, as the second temperature threshold.

In the case described above wherein the element 2 is configured to be activated when a temperature of the inside environment 3 reaches the first threshold temperature from temperatures greater than the first threshold, the element 2 is preferably configured to be activated when a temperature of the inside environment 3 reaches the second threshold temperature from temperatures below the second threshold.

In the latter case, the first threshold is the lower end of the operating range of the electrode E and the second threshold is the upper end of the operating range of the electrode E.

The first threshold may be, for example, zero degrees Celsius, and the second threshold may be, for example, fifty degrees Celsius.

The accompanying drawings also show a cable C connected to the electrode E, which protrudes from the container 1 and which forms a seal with the container 1 in such a way as to prevent the passage of air from the outside environment to the inside environment 3 of the container 1.

The invention described achieves the preset aims, and allow a container to be provided for packaging at least one electrode which guarantees the sterile nature of the electrode and signals to the user if at least one temperature inside the container has left an operating range at least once, or if the temperature has reached at least once one of the ends of that range.

## Claims

1. A sterile container (1) for packaging at least one electrode (E) for medical applications, comprising at least one heat-sensitive element (2) configured to be irreversibly activated when an inside environment (3) of the container reaches at least one temperature threshold, in such a way as to be able to permanently indicate the reaching of this threshold to a user who observes the container from the outside.

2. The container according to claim 1, wherein the element (2) is configured to be activated when reaching the at least one temperature threshold from temperatures lower than the threshold.

3. The container according to claim 1, wherein the element is configured to be activated when reaching the at least one temperature threshold from temperatures higher than the threshold.

4. The container according to claim 1, wherein the element is configured to be irreversibly activated when the inside environment also reaches a further temperature threshold, in such a way as to be able to permanently indicate the reaching of this further threshold to a user who observes the container from the outside.

5. The container according to claim 3 or 4, wherein the element is configured to be activated when reaching the further threshold temperature from temperatures lower than the further threshold.

6. The container according to any one of the preceding claims, wherein the heat-sensitive element comprises ink or paint sensitive to temperature and/or to heat.

7. The container according to any one of the preceding claims, wherein the heat-sensitive element is situated on an inner surface (1a) of the container.

8. The container according to one of claims 1 to 6, wherein the at least one heat-sensitive element (2) is situated on an outer surface of the container.

9. The container according to any one of the preceding claims, wherein the heat-sensitive element forms part of at least a portion of the container configured in such a way that the act of activating or the activation of the element is visible from the outside of the container.

10. The container according to any one of the preceding claims, comprising a plurality of heat-sensitive elements (2) configured to irreversibly activate when the respective predetermined temperature thresholds are reached.

11. A medical apparatus comprising a container according to any one of the preceding claims.
